# EUROPEAN PATENT SPECIFICATION

(11) **EP 1 707 178 B1**
(45) Date of publication and mention of the grant of the patent: **13.07.2011**
(21) Application number: 06005746.0
(22) Date of filing: 21.03.2006
(51) Int. Cl.: A61K 6/10

(54) **Pasty dental alginate impression material composition**
Pastöse Zahnabformmasse auf Alginatbasis
Matériau pâteux pour empreinte dentaire à base de l'alginate

(30) Priority: 28.03.2005 JP 2005090875
(43) Date of publication of application: 04.10.2006
(73) Proprietor: GC Corporation, Tokyo 174-8585 (JP)
(72) Inventor: Kamohara, Hiroshi, C/o GC Corporation, Itabashi-ku Tokyo 174-8585 (JP); Watanabe, Nobutaka, C/o GC Corporation, Itabashi-ku Tokyo 174-8585 (JP)
(74) Representative: Müller-Boré & Partner Patentanwälte

(56) References cited:
- EP-A- 1 510 198
- GB-A- 754 375
- GB-A- 1 073 772
- US-A1- 2002 058 725
- PATENT ABSTRACTS OF JAPAN vol. 2002, no. 07, 3 July 2002 (2002-07-03) -& JP 2002 068919 A (GC CORP), 8 March 2002 (2002-03-08)

## Description

The present invention relates to a pasty dental alginate impression material composition used for obtaining the impressions of an oral cavity in a dentistry, which comprises a base material paste composed mainly of an alginate and water and a setting material paste composed mainly of calcium sulfate and a liquid component which is not reacted with the calcium sulfate. More particularly, even though the pasty dental alginate impression material composition is a mixture made by mixing the same base material paste and the same setting material paste, fluidity of this composition can be changed by changing a mixing ratio of the base material paste and the setting material paste at the time of kneading. Further, this composition can give the sufficient working time, while it has excellent setting ability, furthermore, the setting time is not delayed, and the liquid separation does not occur during storage.

In the dentistry, a dental alginate impression material using alginate has been widely used as the impression material for obtaining the impression of the oral cavity at the time of producing a dental prosthesis. As the dental alginate impression material, the following three types have been used. A first type is made by mixing and kneading water and powders composed mainly of alginate, a gelatinizing reactant, a gelatinizing regulator and a filler, to thereby set those, and a second type is made by mixing and kneading alginate, the gelatinizing regulator, the filler and water with a gelatinizing reactant powder. In addition to these two types, as a third type, a material, which is made by mixing and kneading two kinds of pastes of the base material paste and the setting material paste to thereby cure those, has been also used. In the third type, the pasty dental alginate impression material comprises the base material paste and the setting material paste, where the base material paste is generally made by mainly dissolving alginate with water, and adding a filler powder for giving formability and hardness, and the setting material paste mainly comprises calcium sulfate as a setting material of alginate and the liquid component, which is not reacted with the calcium sulfate such as liquid paraffin.

In these dental alginate impression materials, the powdery dental alginate impression material made by mixing and kneading powders and water is most widely used. When this impression material is used, it is necessary to measure the powders and water and knead mainly by hands. As for this powdery dental alginate impression material, even though it is complicated, fluidity of the mixture can be changed by changing the mixing ratio of the powders and water in accordance with the clinical case or the preference of an operator. This is a frequently using method for daily clinical, and is one reason why the powdery dental alginate impression material is widely used.

On the other hand, as the pasty dental alginate impression material, the following material has been used (for example, refer to Japanese Patent Application Laid Open No. 2002-87922) . It is a material comprising a base material paste and a setting material past, in which the base material paste is composed mainly of alginate, water and filler and the setting material paste is composed mainly of calcium sulfate and a liquid component. The base material paste contains a predetermined amount of specific polysaccharides, and the setting material paste contains a predetermined amount of polybutene. This pasty dental alginate impression material composition can be automatically kneaded by a special machine, so that it becomes to be widely used. Since this pasty dental alginate impression material is a paste-like one, it is easy for kneading. Further, since anyone can obtain the mixture having the fixed quality, there is a remarkable advantage that a problem does not occur at the time of obtaining the impression. Furthermore, since this material can be kneaded continuously by the special machine to be pushed out to a tray for impression directly, the work burden to a dentist or a hygienist can be reduced.

However, as for the pasty dental alginate impression material being used now, the fluidity of the mixture cannot be changed in the same composition even when the mixing ratio of the base material paste and the setting material paste is changed. This is the difference of this material from the powdery dental alginate impression material. Therefore, this material cannot be made in the condition having the suitable fluidity in accordance with the clinical case, and as a result of this, it does not have adaptability capable of using for all clinical cases.

Further, the pasty dental alginate impression material has a problem that the working time is insufficient and an accident in taking impression occurs. The reason why the accident occurs is as follows. If the sufficient working time is kept on clinical, since the pasty dental alginate impression material is set slowly as compared with the powdery dental alginate impression material, the holding time in the mouth becomes long. So, the problem occurs. Furthermore, there is a problem that the fillers in the base material paste are precipitated during storage to thereby make the liquid separation. If the liquid separation occurs, the hardness and the fluidity of a set material are changed during use of it after kneading. As a result of this, the impression becomes easily incomplete.

A primary objective of the present invention is to solve the problems of the pasty dental alginate impression material comprising the base material paste and the setting material paste, which is used now. The problems to be solved are as follows. The fluidity of the mixture cannot be changed even when the mixing ratio is changed, unlike the powdery dental alginate impression material obtained by mixing and kneading the powders and water. Further, the sufficient operation surplus time cannot be kept due to the setting ability, and the liquid separation occurs during storage due to the precipitation of the filler in the base material paste.

The earnest work was carried out in order to solve the above-mentioned problems and, as a result of this, the followings were found out. As for the conventional pasty dental alginate impression material, since the filler is contained in the base material paste composed mainly of alginate and water, the liquid separation occurs during storage due to the precipitation of the filler in the base material paste, and the fluidity of the mixture cannot be changed even when the mixing ratio is changed. Then, the followings were found out to complete the present invention. The filler is removed from the base material paste, and the predetermined amount of the specific polysaccharides is added to the base material paste instead of the filler to thereby regulate the fluidity. Then, the fluidity can be changed by changing the mixing ratio of the base material paste and the setting material paste. Further, since the filler is not contained, the problem that the liquid separation occurs due to the precipitation of the filler can be solved. Furthermore, carbonate of sodium or potassium is used in the base material paste as a retarder, and phosphate of sodium or potassium is used in the setting material paste as a retarder. Then, the problem of the operation surplus time can be solved.

That is, the present invention is a pasty dental alginate impression material composition comprising a base material paste composed mainly of 1-20 % by weight of alginate and water, and a setting material paste composed mainly of 10-60 % by weight of calcium sulfate and a liquid component which is not reacted with the calcium sulfate.

The base material paste does not contain a powdery filler, but contains 0.01-5 % by weight of carbonate of sodium or potassium as a retarder, and 0.01-15 % by weight of one or more polysaccharides selected from carrageenan, pullulan, curdlan, xanthan gum, gellan gum, pectin, konjak glucomannan, xyloglucan, guar gum, gum Arabic and locust bean gum.

The setting material paste contains 0.01-10 % by weight of phosphate of sodium or potassium as a retarder, and 0.5-30 % by weight of polybutene.

Further, the followings were found out. It is preferable that the alginate in the base material paste is one or more kinds of alginate of sodium, potassium, ammonium or triethanolamine. Further, it is preferable that the liquid component, which is not reacted with the calcium sulfate, in the setting material paste is one or more kinds of a group selected from hydrocarbon, fatty alcohol, cyclic alcohol, fatty acid, a salt or an ester of this fatty acid, and a nonaqueous solvent. The hydrocarbon comprises decane, undecane, dodecane, tetradecane, kerosene, 1-octene, cycloheptane, cyclononane, or liquid paraffin. The fatty alcohol comprises 1- hexanol, 1- octanol, citronellol, or oleyl alcohol. The cyclic alcohol comprises benzyl alcohol or meta-cresol . The fatty acid comprises hexanoic acid, octanoic acid, oleic acid or linoleic acid. The nonaqueous solvent comprises polyethyleneglycol or polypropyleneglycol. As for calcium sulfate in the setting material paste, it is preferable to be one or more kinds of anhydrous gypsum, hemihydrate gypsum and dehydrate gypsum. As for polybutene, it is preferable to have number average molecular weight of 300 to 4000. The base material paste may further contain one or more kinds of various inorganic or organic coloring agents, an antiseptic, various disinfectants, and a perfume. Further, the setting material paste may further contain one or more kinds of a pH controlling agent such as magnesium oxide or hydroxide, a surfactant and the fluoride.

The pasty dental alginate impression material composition according to the present invention is used by mixing and kneading the base material paste and the setting material paste, the fluidity can be changed by changing the mixing ratio of the base material paste and the setting material paste, the sufficient working time can be kept while it has sharp setting and, since the filler is contained in the base material paste, the liquid separation due to the precipitation of the filler does not occur during storage. Thus, this pasty dental alginate impression material composition is excellent.

As for the alginate used in the base material paste of the pasty dental alginate impression material composition according to the present invention, one or more kinds of a water-soluble salt can be used, where the salt is, for example, alginate of sodium, potassium, ammonium, triethanolamine or the like. Further, the content of these salts is 1-10 % by weight in the base material paste.

The water used in the base material paste may be ion-exchanged water, distilled water or the like, or may be the water which is disinfected by sodium hypochlorite or the like.

In the base material paste used for the conventional pasty dental alginate impression material, the filler such as silica, diatomaceous earth or the like is contained in order to control the fluidity of the mixture and suitably keep the hardness of the set material. The fluidity of the mixture can be kept in the fixed state by the filler. However, even when the mixing ratio of the base material paste and the setting material paste is changed for changing the fluidity of the mixture, the fluidity of the mixture is hardly changed. So, in order to change the fluidity of the mixture by changing the mixing ratio of the base material paste and the setting material paste, the filler is removed from the base material paste, and one or more kinds of the polysaccharides selected from carrageenan, pullulan, curdlan, xanthan gum, gellan gum, pectin, konjak glucomannan, xyloglucan, guar gum, gum Arabic and locust bean gum are added. Further, 0.01-5 % by weight of carbonate of sodium or potassium is added as the retarder. Then, the fluidity of the mixture can be suitably kept and also can be changed by changing the mixing ratio of the base material paste and the setting material paste in the same composition in accordance with the clinical case or the preference of an operator.

The polysaccharides are stable in an alginate aqueous solution and hardly decomposed, so that the viscosity of the base material paste is hardly decreased. In the polysaccharides, carrageenan is one kind of seaweed polysaccharides and an acid polymer comprising galactose containing a sulfuric acid group or an anhydro group. Further, the pullulan, the curdlan, the xanthan gum and the gellan gum are obtained by separating and refining the polysaccharides produced by a microorganism such as a yeast or the like. In these polysaccharides, the xanthan gum is the most useful and is mucopolysaccharides produced by a germ separated from leaf surface of cabbage. Furthermore, the polysaccharides, such as pectin, konjak glucomannan, xyloglucan, guar gum, gum Arabic and locust bean gum are extracted from a bark, seeds, leaves and stalks, an underground stem or fruit of a plant. It is necessary that 0.01-15 % by weight of one or more kinds of polysaccharides selected from carrageenan, pullulan, curdlan, xanthan gum, gellan gum, pectin, konjak glucomannan, xyloglucan, guar gum, gum Arabic and locust bean gum are contained in the base material paste composed mainly of the alginate and water. If the content is less than 0.01 % by weight, the viscosity of the base material paste is too low, so that it can not be used as the impression material. If content is more than 15 % by weight, the viscosity becomes too high, so that it is hardly kneaded to cause the problem in the taking impression operation. Preferably, the content is 0.1 to 3 % by weight.

As for calcium sulfate used in the setting material paste of the pasty dental alginate impression material composition according to the present invention, more particularly, the anhydrous gypsum, the hemihydrate gypsum, the dehydrate gypsum or the like can be used.

As the liquid component which is not reacted with the calcium sulfate used in the setting material paste, the hydrocarbon, a fatty alcohol, a cyclic alcohol, a fatty acid, a salt or an ester of this fatty acid, and a nonaqueous solvent can be used. More particularly, the hydrocarbon such as decane, undecane, dodecane, tetradecane, kerosene, 1-octene, cycloheptane, cyclononane or liquid paraffin, the fatty alcohol such as 1- hexanol, 1- octanol, citronellol or oleyl alcohol, the cyclic alcohol such as benzyl alcohol or meta-cresol, the fatty acid such as hexanoic acid, octanoic acid, oleic acid or linoleic acid, a salt or an ester of these fatty acids and the nonaqueous solvent such as polyethyleneglycol or polypropyleneglycol are suitably used.

As for the polybutene used in the setting material paste, when the phosphate of sodium or potassium described below is added, the curing ability is increased and the sufficient working time can be kept. Further, polybutene prevents occurrence of the liquid separation due to the precipitation of the calcium sulfate in the setting material paste, so that it can prevent decrease of the setting ability. The polybutene is a liquid polymer where isobutylene as a base material and a few 1-butene are copolymerized. Polybutene having the number average molecular weight of 300 to 4000 is generally used. It is necessary that 0.5-30 % by weight of the polybutene is contained in the setting material paste. If the content is less than 0.5 % by weight, the liquid component is separated. If the content is more than 30 % by weight, the setting time becomes remarkably long under coexistence of the phosphate of sodium or potassium, and the hardness of the set material is decreased. Preferably, the content is 0.5 to 15 % by weight.

In order to obtain the excellent setting ability, the pasty dental alginate impression material composition according to the present invention contains 0.01-5 % by weight of carbonate of sodium or potassium in the base material paste, and 0.01-10 % by weight of phosphate of sodium or potassium in the setting material paste, where the base material paste is composed mainly of 1-20 % by weight of alginate and water, and the setting material paste is composed mainly of 10-60 % by weight of calcium sulfate and the liquid component which is not reacted with the calcium sulfate. By this combination, the sharp setting ability can be obtained while the sufficient working time is obtained. On the contrary, when the phosphate of sodium or potassium is contained in the base material paste and the carbonate of sodium or potassium is contained in the setting material paste, the above effects cannot be obtained.

If blending amounts of the carbonate of sodium or potassium and the phosphate of sodium or potassium are less than 0.01 % by weight with respect to each paste, the setting time is too short. Further, if the carbonate of sodium or potassium is more than 5 % by weight or the phosphate of sodium or potassium is more than 10 % by weight, the working time is too long. Thus, in both cases, these pastes cannot be used as the dental impression material.

The pasty dental alginate impression material composition according to the present invention may, in the range in which the characteristics are not lost, contain various kinds of inorganic or organic coloring agents, an antiseptic, various kinds of disinfectants, a perfume or the like, in the base material paste thereof . Further, it may contain magnesium oxide, a pH controlling agent such as magnesium oxide or hydroxide or the like, a surfactant for increasing the mixing ability with the base material paste, a fluoride for improving a model surface.

### [Example]

Hereinafter, the present invention is described concretely with examples, but the present invention is not limited to these examples.

### Example 1

| Base material paste | |
|---|---|
| Sodium alginate | 13 (% by weight) |
| Distilled water | 74.98 |
| Locust bean gum | 12 |
| Sodium carbonate | 0.02 |

| Setting material paste | |
|---|---|
| Calcium sulfatehemihydrate | 50 (% by weight) |
| Liquid paraffin | 49 |
| Polybutene | 0.5 |
| (a number average molecular weight: 3900) Sodium phosphate | 0.5 |

Each of the above pastes was sufficiently mixed by a mixer to thereby obtain a pasty dental alginate impression material composition. Then, the fluidity was measured by the steps of kneading the base material paste and the setting material paste at the volume ratio of 1 : 1 and 3 : 1; inserting a pushing out rod into a pushing out ring from the lower side to the position of the reference line, filling the kneaded sample in the pushing out ring; eliminating an excess heaped above the upper surface of the pushing out ring by a spatula; reversing the pushing out ring after 30 seconds; pushing out the sample gradually and perpendicularly on an acrylics board by the pushing out rod; measuring a maximum part and a minimum part of a spread of the sample by a caliper when it isgelled; and thereby measuring the fluidity from an average of the measured maximum parts and minimum parts. The pushing out rod had the outer diameter of 35 mm, a reference line on the position of 20 mm from the upper side, and a cellophane film adhered thereon. The pushing out ring had the inner diameter of 35 mm and the height of 50 mm. Then, in each ratio, the working time was measured according to the ISO 1563, and the setting time (the gelling time) was measured according to the ISO 1563. After the base material paste was filled in an aluminum packaging material and kept at 60°C for one week, a state of the liquid separation was observed visually. These results were shown in Table 1 collectively.

### Example 2

| Base material paste | |
|---|---|
| Sodium alginate | 10 (% by weight) |
| Distilled water | 89.78 |
| Konjak glucomannan | 0.02 |
| Sodium carbonate | 0.2 |

| Setting material paste | |
|---|---|
| Calcium sulfate hemihydrate | 60 (% by weight) |
| Liquid paraffin | 5 |
| Polybutene | 28 |
| (a number average molecular weight: 390) | |
| Sodium phosphate | 7 |

Each of the above pastes was sufficiently mixed by a mixer to thereby obtain a pasty dental alginate impression material composition. The same test as that of Example 1 was carried out, and these results were shown in Table 1.

### Example 3

| Base material paste | |
|---|---|
| Sodium alginate | 7 (% by weight) |
| Distilled water | 91.3 |
| Xanthan gum | 1.5 |
| Sodium carbonate | 0.2 |

| Setting material paste | |
|---|---|
| Calcium sulfatehemihydrate | 50 (% by weight) |
| Liquid paraffin | 47 |
| Polybutene | 1 |
| (a number average molecular weight: 1400) | |
| Sodium pyrophosphate | 2 |

Each of the above pastes was sufficiently mixed by a mixer to thereby obtain a pasty dental alginate impression material composition. The same test as that of Example 1 was carried out, and these results were shown in Table 1.

### Example 4

| Base material paste | |
|---|---|
| Sodiumalginate | 17 (% by weight) |
| Distilled water | 81.97 |
| Xanthan gum | 1 |
| Sodium hypochlorite | 0.01 |
| Sodium carbonate | 0.02 |

| Setting material paste | |
|---|---|
| Calcium sulfate hemihydrate | 50 (% by weight) |
| Liquid paraffin | 33 |
| Polybutene | 2 |
| (a number average molecular weight: 1400) | |
| Fluoride potassium titanate | 5 |
| Magnesium hydroxide | 5 |
| Polyoxyethylene alkylether | 2 |
| Sodium pyrophosphate | 3 |

Each of the above pastes was sufficiently mixed by a mixer to thereby obtain a pasty dental alginate impression material composition. The same test as that of Example 1 was carried out, and these results were shown in Table 1.

### Comparison example 1

| Base material paste | |
|---|---|
| Sodium alginate | 10 (% by weight) |
| Diatomaceous earth | 20 |
| Distilled water | 70 |

| Setting material paste | |
|---|---|
| Calcium sulfate hemihydrate | 50 (% by weight) |
| Liquid paraffin | 50 |

Each of the above pastes was sufficiently mixed by a mixer to thereby obtain a pasty dental alginate impression material composition. The same test as that of Example 1 was carried out, and these results were shown in Table 1.

### Comparison example 2

| Base material paste | |
|---|---|
| Sodium alginate | 7 (% by weight) |
| Diatomaceous earth | 20 |
| Distilled water | 71 |
| Xanthan gum | 2 |

| Setting material paste | |
|---|---|
| Calciumsulfatehemihydrate | 50 (% by weight) |
| Liquid paraffin | 49.5 |
| Polybutene | 0.5 |

(a number average molecular weight: 1400)

Each of the above pastes was sufficiently mixed by a mixer to thereby obtain a pasty dental alginate impression material composition. The same test as that of Example 1 was carried out, and these results were shown in Table 1.

**Table 1**

| | Example 1 | | Example 2 | | Example 3 | | Example 4 | | Comparison example 1 | | Comparison example 2 | |
|---|---|---|---|---|---|---|---|---|---|---|---|---|
| Fluidity (mm) | 45 | 55 | 42 | 53 | 43 | 55 | 42 | 53 | 42 | 43 | 43 | 45 |
| Working Time (minute: second) | 1:05 | 2:05 | 0:55 | 1:50 | 1:00 | 2:00 | 1:10 | 2:05 | 0:45 | 1:10 | 0:40 | 1:05 |
| Setting Time (minute:second) | 1:30 | 2:30 | 1:20 | 2:20 | 1:20 | 2:20 | 1:30 | 2:20 | 1:30 | 2:10 | 1:30 | 2:00 |
| Liquid Separation (Storage Stability) | No liquid separation | | No liquid separation | | No liquid separation | | No liquid separation | | Liquid separation occurred at the upper part of both pastes. | | Liquid separation occurred at the upper part of the base material paste. | |

As clearly known from the Table 1, in the pasty dental alginate impression material composition according to the present invention, the fluidity can be changed by changing the mixing ratio of the base material paste composed mainly of the alginate and water and the setting material paste composed mainly of the calcium sulfate and the liquid component which is not reacted with the calcium sulfate, when the base material paste and the setting material paste are mixed and kneaded, while the setting time is not largely changed, the sufficient working time can be kept, and the liquid separation does not occur in the setting paste material and base material paste even when it is kept at 60°C for one week.

## Claims

1. A pasty dental alginate impression material composition, comprising a base material paste composed mainly of 1-20 % by weight of alginate and water, and a setting material paste composed mainly of 10-60 % by weight of calcium sulfate and a liquid component which is not reacted with the calcium sulfate, wherein said base material paste does not contain a filler, but contains 0.01-5 % by weight of carbonate of sodium or potassium as a retarder, and 0.01-15 % by weight of one or more polysaccharides selected from carrageenan, pullulan, curdlan, xanthan gum, gellan gum, pectin, konjak glucomannan, xyloglucan, guar gum, gum Arabic and locust bean gum, and
wherein said setting material paste contains 0.01-10 % by weight of phosphate of sodium or potassium as a retarder, and 0.5-30 % by weight of polybutene.

2. The pasty dental alginate impression material composition as claimed in claim 1,
wherein the alginate in the base material paste is one or more kinds of a alginate of sodium, potassium,
ammonium or triethanolamine.

3. The pasty dental alginate impression material composition as claimed in claim 1 or 2,
wherein the liquid component, which is not reacted with the calcium sulfate, in the setting material paste is one or more kinds of a group selected from hydrocarbon, fatty alcohol, cyclic alcohol, a fatty acid, a salt or an ester of this fatty acid, and a nonaqueous solvent, wherein the hydrocarbon comprises decane, undecane, dodecane, tetradecane, kerosene, 1-octene, cycloheptane, cyclononane, or liquid paraffin, wherein the fatty alcohol comprises 1- hexanol, 1-octanol, citronellol, or oleyl alcohol,
wherein the cyclic alcohol comprises benzyl alcohol or meta-cresol,
wherein the fatty acid comprises hexanoic acid, octanoic acid, oleic acid or linoleic acid, and wherein the nonaqueous solvent comprises polyethyleneglycol or polypropyleneglycol.

4. The pasty dental alginate impression material composition as claimed in any one of claims 1 to 3, wherein the calcium sulfate in the setting material paste is one or more kinds of anhydrous gypsum, hemihydrate gypsum and dehydrate gypsum.

5. The pasty dental alginate impression material composition as claimed in any one of claims 1 to 4, wherein the number average molecular weight of the polybutene is 300 to 4000.

6. The pasty dental alginate impression material composition as claimed in any one of claims 1 to 5, wherein one or more kinds of various inorganic or organic coloringagents, anantiseptic, various disinfectants, and a perfume are further contained in the base material paste.

7. The pasty dental alginate impression material composition as claimed in any one of claims 1 to 6, wherein one or more kinds of a pH controlling agent such as magnesium oxide or hydroxide, a surfactant and the fluoride are further contained in the setting material agent.

## Patentansprüche

1. Pastöse Alginat-Zahnabformmaterialzusammensetzung, umfassend eine Basismaterialpaste, die vorwiegend aus 1 bis 20 Gew.-% Alginat und Wasser zusammengesetzt ist, und eine Härtungsmaterialpaste, die vorwiegend aus 10 bis 60 Gew.-% Calciumsulfat und einer flüssigen Komponente, die nicht mit dem Calciumsulfat umgesetzt wird, zusammengesetzt ist, wobei die Basismaterialpaste keinen Füllstoff, sondern 0,01 bis 5 Gew.-% eines Carbonats von Natrium oder Kalium als Verzögerungsmittel und 0,01 bis 15 Gew.-% eines Polysaccharids oder mehrerer Polysaccharide enthält, das oder die aus Karrageen, Pullulan, Curdlan, Xanthangummi, Gellangummi, Pektin, Konjak-Glukomannan, Xyloglukan, Guargummi, Gummiarabikum und Johannisbrotkernmehl ausgewählt ist oder sind, und
wobei die Härtungsmaterialpaste 0,01 bis 10 Gew.-% eines Phosphats von Natrium oder Kalium als Verzögerungsmittel und 0,5 bis 30 Gew.-% Polybuten enthält.

2. Pastöse Alginat-Zahnabformmaterialzusammensetzung nach Anspruch 1, bei der das Alginat in der Basismaterialpaste eine Art oder mehrere Arten eines Alginats von Natrium, Kalium, Ammonium oder Triethanolamin ist.

3. Pastöse Alginat-Zahnabformmaterialzusammensetzung nach Anspruch 1 oder 2, bei der die flüssige Komponente, die nicht mit dem Calciumsulfat umgesetzt wird, in der Härtungsmaterialpaste eine Art oder mehrere Arten von einer Gruppe, ausgewählt aus Kohlenwasserstoff, Fettalkohol, cyclischem Alkohol, einer Fettsäure, einem Salz oder einem Ester dieser Fettsäure und einem nicht-wässrigen Lösungsmittel ist, wobei der Kohlenwasserstoff Decan, Undecan, Dodecan, Tetradecan, Kerosin, 1-Octen, Cycloheptan, Cyclononan oder flüssiges Paraffin umfasst, wobei der Fettalkohol 1-Hexanol, 1-Octanol, Citronellol oder Oleylalkohol umfasst, wobei der cyclische Alkohol Benzylalkohol oder meta-Kresol umfasst, wobei die Fettsäure Hexansäure, Octansäure, Ölsäure oder Linolsäure umfasst, und wobei das nichtwässrige Lösungsmittel Polyethylenglykol oder Polypropylenglykol umfasst.

4. Pastöse Alginat-Zahnabformmaterialzusammensetzung nach einem der Ansprüche 1 bis 3, bei der das Calciumsulfat in der Härtungsmaterialpaste eine oder mehrere Arten von wasserfreiem Gips, Hemihydrat-Gips und Gips ist.

5. Pastöse Alginat-Zahnabformmaterialzusammensetzung nach einem der Ansprüche 1 bis 4, bei der das Zahlenmittel des Molekulargewichts des Polybutens 300 bis 4000 beträgt.

6. Pastöse Alginat-Zahnabformmaterialzusammensetzung nach einem der Ansprüche 1 bis 5, bei der in der Basismaterialpaste ferner eine Art oder mehrere Arten verschiedener anorganischer oder organischer Farbmittel, eines Antiseptikums, verschiedener Desinfektionsmittel und eines Parfüms enthalten ist oder sind.

7. Pastöse Alginat-Zahnabformmaterialzusammensetzung nach einem der Ansprüche 1 bis 6, bei der in dem Härtungsmaterialmittel ferner eine Art oder mehrere Arten eines pH-Einstellmittels, wie Magnesiumoxid oder -hydroxid, eines grenzflächenaktiven Mittels und eines Fluorids enthalten ist oder sind.

## Revendications

1. Une composition de matériau pâteux pour empreinte dentaire à base d'alginate, comprenant, comme matériau de base, une pâte composée principalement de 1-20% en poids d'alginate et d'eau, et une pâte servant de matériau de durcissement composée principalement de 10-60% en poids de sulfate de calcium et un composant liquide qui n'est pas mis en réaction avec le sulfate de calcium,
dans laquelle ladite pâte servant de matériau de base ne contient pas de charge, mais contient 0,01-5% en poids de carbonate de sodium ou de potassium en tant que retardateur, et 0,01-15% en poids d'un ou plusieurs polysaccharides sélectionnés parmi la carraghénine, le pullulane, le curdlane, la gomme de xanthane, la gomme gellane, la pectine, le glucomannane de konjak, le xyloglucane, la gomme de guar, la gomme arabique et la gomme de caroube, et
dans laquelle ladite pâte servant de matériau de durcissement contient 0,01-10% en poids de phosphate de sodium ou de potassium en tant que retardateur, et 0,5-30% en poids de polybutène.

2. La composition de matériau pâteux pour empreinte dentaire à base d'alginate telle que revendiquée dans la revendication 1,
dans laquelle l'alginate dans la pâte servant de matériau de base est une ou plusieurs sortes d'un alginate de sodium, de potassium, d'ammonium ou de triéthanolamine.

3. La composition de matériau pâteux pour empreinte dentaire à base d'alginate telle que revendiquée dans la revendication 1 ou la revendication 2,
dans laquelle le composant liquide, qui n'est pas mis en réaction avec le sulfate de calcium, dans la pâte servant de matériau de durcissement est une ou plusieurs sortes d'un groupe sélectionné parmi de l'hydrocarbure, de l'alcool gras, de l'alcool cyclique, un acide gras, un sel ou un ester de cet acide gras, et un solvant non aqueux,
dans laquelle l'hydrocarbure comprend du décane, de l'undécane, du dodécane, du tétradécane, du kérosène, du 1-octène, du cycloheptane, du cyclononane, ou de la paraffine liquide,
dans laquelle l'alcool gras comprend du 1-hexanol, du 1-octanol, du citronellol ou de l'alcool oléylique,
dans laquelle l'alcool cyclique comprend de l'alcool de benzyle ou du métacrésol, dans laquelle l'acide gras comprend de l'acide hexanoïque, de l'acide octanoïque, de l'acide oléique ou de l'acide linoléique, et
dans laquelle le solvant non aqueux comprend du polyéthylèneglycol ou du polypropylèneglycol.

4. La composition de matériau pâteux pour empreinte dentaire à base d'alginate telle que revendiquée dans l'une quelconque des revendications 1 à 3, dans laquelle le sulfate de calcium dans la pâte servant de matériau de durcissement est une ou plusieurs sortes d'anhydrite, de gypse semi-hydraté et de gypse déshydraté.

5. La composition de matériau pâteux pour empreinte dentaire à base d'alginate telle que revendiquée dans l'une quelconque des revendications 1 à 4, dans laquelle la masse moléculaire moyenne en nombre du polybutène est de 300 à 4000.

6. La composition de matériau pâteux pour empreinte dentaire à base d'alginate telle que revendiquée dans l'une quelconque des revendications 1 à 5, dans laquelle la pâte servant de matériau de base contient en outre une ou plusieurs sortes de divers colorants, un antiseptique, divers désinfectants et un parfum.

7. La composition de matériau pâteux pour empreinte dentaire à base d'alginate telle que revendiquée dans l'une quelconque des revendications 1 à 6, dans laquelle l'agent servant de matériau de durcissement contient en outre une ou plusieurs sortes d'un agent de contrôle du pH comme de l'oxyde ou de l'hydroxyde de magnésium, un tensio-actif et du fluorure.
